# EUROPEAN PATENT APPLICATION

(11) **EP 3 290 431 A1**
(43) Date of publication of application: **07.03.2018**
(21) Application number: 16786582.3
(22) Date of filing: 28.04.2016
(51) Int. Cl.: C07K 1/16, C07K 1/14, C07K 1/18, C07K 1/22

(54) **PROTEIN PURIFICATION METHOD**

(30) Priority: 30.04.2015 JP 2015093241
(71) Applicant: Showa Denko K.K., Tokyo 105-8518 (JP)
(72) Inventor: MATSUI, Natsuno, Tokyo 105-8518 (JP); KOKIDO, Yuzuru, Tokyo 105-8518 (JP); AOKI, Hirobumi, Tokyo 105-8518 (JP); YONEDA, Tadashi, Tokyo 105-8518 (JP)
(74) Representative: Strehl Schübel-Hopf & Partner
(86) International application number: PCT/JP2016/063419
(87) International publication number: WO 2016/175306

(57) **Abstract**

An object of the present invention is to obtain a protein monomer useful as a raw material for medicines industrially and economically in high yield and high purity. In the method for purifying a protein of the present invention, a protein solution containing a protein monomer and a protein aggregate is passed through a column holding a porous rigid polymeric self-supporting structure to which a hydrophobic group is immobilized, and then recovering the protein monomer in a flow-through mode.

## Description

### TECHNICAL FIELD

The present invention relates to a protein purification method that removes protein aggregates and / or host cell proteins from a solution containing protein monomers and protein aggregates and / or host cell proteins in a biopharmaceutical manufacturing process.

Priority is claimed based on Japanese Patent Application No. 2015-093241 filed in Japan on April 30, 2015, the contents of which are incorporated herein by reference.

### BACKGROUND ART

In the process of manufacturing biopharmaceuticals, proteins form aggregates such as dimers and trimers, and the formed protein aggregates sometimes become impurities derived from the objective substance. It is known that proteins associate between molecules in the manufacturing process (concentration, acidic pH exposure, heating operation) and in the storage process (solution, frozen solution, lyophilization) to form aggregates. It is feared that the aggregates have a harmful effect on pharmaceuticals, such as decrease in efficacy and development of immunogenicity. Therefore, it is desired to remove aggregates, which are impurities, to such an extent that there is no harmful effect on a human body.

Accordingly, many methods for removing aggregates by chromatography have been developed with the objective of purifying medicinal proteins, particularly antibody proteins and the like.

Methods reported include those using one of anion exchange chromatography, cation exchange chromatography, and hydrophobic chromatography alone, using several of them together, or using a mixed mode chromatography in which these are mixed. All of these are based on chromatography using a bead-shaped porous polymer carrier. In order to separate them due to a slight difference in interaction between protein aggregates and protein monomers, it is required to precisely control the separation conditions and to exhibit excellent resolution performance. However, it is known that in a chromatography using a bead-shaped porous polymer carrier, the separation performance decreases at a high flow rate.

A method of separating a solution of an antibody containing an aggregate by gel filtration chromatography by containing arginine or an arginine derivative in the solution is also reported. The protein diffuses into the stationary phase space of the column packing material, and elutes according to its molecular weight. It is possible to separate protein aggregates and protein monomers from the difference in elution order, but the elution order takes time because it depends on diffusion. It is necessary to incorporating the step of adding arginine to the solution into the purification process and it is possible that a step of removing arginine become an obliging step in the future. These methods are difficult to realize shortening of the purification process time by rapid separation by rapid passage at high flow rate.

As a method for removing protein aggregates at a high flow rate, a method for purifying antibodies using a porous membrane having anion exchange groups immobilized therein is disclosed (Patent Document 1). However, in the disclosed method, a 2 mL hollow porous membrane module is passed at a flow rate of 2 mL/min. This does not change the flow rate per volume from 1 mL/min flow rate used for commercially available bead-filled type anion exchange chromatography (column solution 1 mL). Therefore, rapid separation due to high flow rate flow has not been realized, and a new method is demanded.

Purification of antibodies from protein mixtures using hydrophobic interaction chromatography (HIC) in flow-through mode is also known (Patent Document 2). However, in the disclosed method, an anion exchange chromatography (AEX) and a HIC are continuously used. In the process using AEX, impurities such as DNA, host cell protein, protein A, virus and protein medium components are mainly removed. In the process using HIC, protein aggregates are mainly removed. However, whether HIC can be used alone is not described in detail. Therefore, processes that can use HIC are limited, and a new method using HIC arbitrarily is desired.

An affinity chromatography method for the purification of polypeptides containing histidine tags by elution of the polypeptides from the chromatography material with solutions containing imidazole or imidazole derivatives are also known (Patent Document 3). However, the disclosed method is limited to a polypeptide containing a histidine tag, and a purification method of various protein aggregates used for biopharmaceuticals is demanded.

In the method of purifying a solution containing at least one of an antibody containing a protein aggregate, a hydrophobic protein or a hydrophobic peptide by gel filtration chromatography, a method for increasing the recovery rate of an antibody containing a protein aggregate, a hydrophobic protein or a hydrophobic peptide by adding arginine or an arginine derivative in a developing solvent are also known (Patent Document 4). However, since the disclosed method requires a step of adding arginine, and there is a demand for processing antibodies containing protein aggregates, hydrophobic proteins or hydrophobic peptides by a simpler operation.

Purification of antibody protein aggregates using a temperature responsive cationic ion exchange resin has also been reported (Patent Document 5). However, since the disclosed method is 0.4 mL/min, the purification process takes time. Purification at a higher flow rate is required.

Aggregate purification of antibody proteins using membranes of disposable materials made from polyamides has also been reported (Patent Document 6). However, the disclosed method is purification method for 1 ml human immunoglobulin G dissolved at 2 mg/mL, which is far from the industrial scale. There is a demand for a method for realizing removal of protein aggregates more industrially.

Methods for removing immunoglobulin aggregates and immunoglobulin fragments using anion exchange chromatography materials have also been reported (Patent Document 7). However, in the disclosed method, the anion exchange chromatography step has to be carried out within a narrow pH range from 7.8 to 8.8. A method capable of removing protein aggregates at a wide pH value range is required.

In addition, a host cell protein (HCP) is characteristic as impurities of biopharmaceuticals. Heterocellular proteins are antigenic to humans, and HCP from mammalian cells has a tumorigenic risk.

Biopharmaceuticals are normally manufactured using mammalian cells, insect cells, Escherichia coli or the like. By contamination of impurities of the protein / peptide derived from the host cells, so-called HCP, at the time of manufacture, it is known that a serious immune reaction against HCP and an increase in immunogenicity of the main agent (an adjuvant action of HCP) are caused in the patient to whom the drugs were administered. Therefore, in order to minimize the contamination of HCP in biopharmaceuticals, it is very important to evaluate the clearance capacity of HCP in each purification step using appropriate analytical techniques.

Based on this, many methods for removing HCP and protein aggregates by chromatography have been developed for the purpose of purifying medicinal proteins, particularly antibody proteins and the like.

Reported methods of removing HCP include hydrophobic interaction chromatography, cation exchange chromatography, anion exchange chromatography, hydroxyapatite chromatography, protein A affinity chromatography, multimodal chromatography and the like. All of these are based on chromatography using a bead-shaped porous polymer carrier. In order to purify HCP, it is required to precisely control the separation conditions and to exhibit excellent resolution performance. However, it is known that in a chromatography using a bead-shaped porous polymer carrier, the separation performance decreases at a high flow rate.

Purification of antibodies from protein mixtures using hydrophobic interaction chromatography (HIC) in flow-through mode is also known (Patent Document 2). However, in the disclosed method, an anion exchange chromatography (AEX) and a HIC are continuously used. In the process using AEX, impurities such as DNA, host cell protein, protein A, virus and protein medium components are mainly removed. In the process using HIC, protein aggregates are mainly removed. However, it is not mentioned whether HCP and protein aggregates can be removed at the same time. Therefore, a new method for purifying a protein that can simultaneously remove both of HCP and protein aggregates is desired.

It is known that removal of HCP is performed by protein A chromatography or the like (Patent Document 8). However, the disclosed method separates only HCP and does not mention whether separating protein aggregates can be performed at the same time. Therefore, a method for purifying a new protein that can simultaneously remove HCP and protein aggregates is desired.

It is known that acidic proteins are purified by ceramics hydroxyapatite chromatography (Patent Document 9). However, although it is possible to remove protein aggregates and HCP at the same time in the disclosed method, it takes 800 minutes to regenerate the column for its separation. Therefore, there is a need for a new method for purifying a protein that can simultaneously remove HCP and protein aggregates at high speed.

[Patent Document 1] Japanese Unexamined Patent Application, First Publication No. 2010-241761
[Patent Document 2] Published Japanese Translation No. 2013-519652 of PCT International Application
[Patent Document 3] Published Japanese Translation No. 2013-527851 of PCT International Application
[Patent Document 4] Japanese Unexamined Patent Application, First Publication No. 2006-242957
[Patent Document 5] Japanese Unexamined Patent Application, First Publication No. 2014-129319
[Patent Document 6] Japanese Unexamined Patent Application, First Publication No. 2005-145852
[Patent Document 7] Published Japanese Translation No. 2012-513425 of PCT International Application
[Patent Document 8] Published Japanese Translation No. 2013-517318 of PCT International Application
[Patent Document 9] Published Japanese Translation No. 2012-507550

### SUMMARY OF THE INVENTION

The present invention has been made in view of the above circumstances, and it is an object of the present invention to obtain a protein monomer useful as a raw material for medicines industrially and economically with high yield and high purity. More specifically, a method for rapidly removing protein aggregates and / or HCP from a mixture containing protein monomers and protein aggregates and / or HCP at high speed to obtain protein monomers in high yield and a method for purifying a protein which makes it possible to shorten the time of the considering condition for selectively recovering protein monomers.

### SUMMARY OF THE INVENTION

The present inventors diligently studied methods of purifying protein aggregates and / or proteins to be removed by HCP. As a result, it was found that protein monomers can be recovered under high flow rate conditions when chromatography is performed using a column holding a porous rigid polymeric self-supporting structure to which a hydrophobic group is immobilized. In addition, using this knowledge, it was found that examination of conditions for selectively recovering protein monomers can shorten the time of condition examination, and the present invention was accomplished.

The present invention is as follows.
[1] A protein purification method, including a step of recovering a protein monomer in a flow-through mode by passing a protein solution, which contains the protein monomer and a protein aggregate, through a column holding a porous rigid polymeric self-supporting structure to which a hydrophobic group is immobilized.
[2] The purification method according to [1], including steps of loading a protein monomer and a protein aggregate by passing a solution, which contains the protein monomer and the aggregate, through a column holding a porous rigid polymeric self-supporting structure to which a hydrophobic group is immobilized, and
   recovering the protein monomer in a flow-through mode by absorbing the aggregate by passing a mobile phase through the column loaded with the protein monomer and the protein aggregate.
[3] A purification method according to [1], including a step of recovering the protein monomer in a flow-through mode by absorbing the aggregate in the column by passing a protein solution, which contains a protein monomer and protein aggregates, through a column holding a porous rigid polymeric self-supporting structure to which a hydrophobic group is immobilized.
[4] The purification method according to [1], wherein the protein solution further contains a host cell protein (HCP).
[5] The purification method according to [4], including steps of loading a protein monomer, the aggregate and a host cell protein (HCP) by passing a solution, which contains the protein monomer, the aggregate and a host cell protein (HCP), through a column holding a porous rigid polymeric self-supporting structure to which a hydrophobic group is immobilized, and
   recovering the protein monomer in a flow-through mode by absorbing the aggregate and a host cell protein (HCP) by passing a mobile phase through the column loaded with the protein monomer, the aggregate and a host cell protein (HCP).
[6] The purification method according to [4], including a step of recovering the protein monomer in a flow-through mode by absorbing the aggregate and the host cell protein (HCP) in the column by passing a protein solution, which contains a protein monomer, the aggregate and a host cell protein (HCP), through a column holding a porous rigid polymeric self-supporting structure to which a hydrophobic group is immobilized.
[7] The purification method according to any one of [1] to [6], wherein the monomer constituting the porous rigid polymeric self-supporting structure is a methacrylate or an acrylate.
[8] The purification method according to any one of [1] to [7], wherein the thickness of the porous rigid polymeric self-supporting structure in the liquid flowing direction is 1 to 100 mm.
[9] The purification method according to any one of [1] to [8], wherein the flow rate of the mobile phase is 2 CV/min or more.
[10] The purification method according to any one of [1] to [9], wherein the concentration of the protein contained in the protein solution is 0.001 to 20 mg/mL.
[11] The purification method according to any one of [1] to [10], wherein the protein loading amount per 1 mL of the column volume of the column is 0.001 to 50 mg.
[12] The purification method according to any one of claims [1] to [11], further including a preliminary purification step using an ion exchange chromatography, and an affinity chromatography, and
   the purification method is carried out after the preliminary purification step.

### EFFECT OF THE INVENTION

According to the protein purification method for removing the protein aggregate and / or HCP of the present invention, a protein monomer useful as a raw material for medicines and the like can be industrially and economically obtained in high yield and high purity. More specifically, protein aggregates and / or HCPs can be removed at high speed from a mixture containing protein monomers and protein aggregates and / or HCPs to obtain protein monomers in high yield. Further, by examining conditions for selectively recovering protein monomers using this knowledge, it is possible to shorten the time of condition examination.

### BRIEF DESCRIPTION OF THE DRAWINGS

FIG. 1 shows the flow-through portion in the chromatogram.
FIG. 2 shows a flow path diagram of an apparatus used for calculating a recovery rate of an embodiment.
FIG. 3 shows a flow path diagram of an apparatus used for purity calculation of an example.

### DETAIL DESCRIPTION OF THE INVENTION

Hereinafter, preferred embodiments for carrying out the present invention will be described. It is to be noted that the embodiment described below represents one example of a representative embodiment of the present invention, and the present invention is not limited thereto, so that the scope of the present invention is not interpreted narrowly by them.

### (First Embodiment: Two-Step Purification)

### [Method for Removing Protein Aggregate]

The method for removing protein aggregates according to the first embodiment of the present invention includes steps of loading a protein monomer and a protein aggregate by passing a solution, which contains the protein monomer and the aggregate, through a column holding a porous rigid polymeric self-supporting structure to which a hydrophobic group is immobilized, and recovering the protein monomer in a flow-through mode by absorbing the aggregate by passing a mobile phase through the column loaded with the protein monomer and the protein aggregate.

### [Removal method of protein aggregate and HCP]

The method for removing protein aggregates and HCP according to the first embodiment of the present invention includes steps of loading a protein monomer, the aggregate and a host cell protein (HCP) by passing a solution, which contains the protein monomer, the aggregate and a host cell protein (HCP), through a column holding a porous rigid polymeric self-supporting structure to which a hydrophobic group is immobilized, and recovering the protein monomer in a flow-through mode by absorbing the aggregate and the host cell protein (HCP) by passing a mobile phase through the column loaded with the protein monomer, the aggregate and a host cell protein (HCP).

Further, the method for purifying a protein according to the first embodiment of the present invention may further include a preliminary purification step by ion exchange chromatography or affinity chromatography, prior to the protein purification process.

### <Hydrophobic group>

Examples of the hydrophobic group include a normal-aliphatic group, an iso-aliphatic group, a neo-aliphatic group, and an oligo (alkylene glycol) group. Specific examples thereof include propyl group, butyl group, pentyl group, hexyl group, heptyl group, octyl group, octadecyl group, docosyl group, octacosyl group, poly (ethylene glycol) group, poly (propylene glycol) group, phenyl group and the like. They may be linked to other functional groups by a covalent bond.

The hydrophobic group is preferably a butyl group, an octyl group, a phenyl group, an octadecyl group, a docosyl group, an octacosyl group, more preferably a butyl group, an octyl group, a phenyl group, or most preferably a butyl group.

### <Porous rigid polymeric self-supporting structure>

The rigid porous polymeric self-supporting structure means a polymeric structure obtained by polymerization of a monomer having at least two or more polymerizable moieties, or at least two kinds of monomers (the first type of monomer has a polymerizable part, and another type of monomer is capable of crosslinking the polymer chain obtained by polymerization of the first monomer). It is also porous and the pores have a uniform pore size distribution throughout. The shape is formed into an integral structure such as a plate shape, a tubular shape, a cylindrical shape, and the like.

The thickness of the porous rigid polymeric self-supporting structure in the liquid flowing direction is preferably 1 to 100 mm, more preferably 2 to 70 mm, most preferably 3 to 60 mm, further preferably 3 to 50 mm.

By setting the thickness of the porous rigid polymeric self-supporting structure to 1 mm or more, it has sufficient mechanical strength.

By setting the thickness of the porous rigid polymeric self-supporting structure to 100 mm or less, it is possible to keep the pressure at which the rigid porous polymeric self-supporting structure does not break and to prevent the pump pressure from rising.

As the monomer constituting the porous rigid polymeric self-supporting structure, a monomer having a hydrophobic group is used, and for example, a polyvinyl monomer and a monovinyl monomer can be used.

Examples of the polyvinyl monomer include vinyl ethers such as divinylbenzene, divinylnaphthalene, divinylpyridine, methacrylates, acrylates, vinyl esters and divinyl ether, alkylene bisacrylamides; or methacrylamides such as ethylenebisacrylamide and propylenebisacrylamide, can be used.

Examples of the methacrylates include glycidyl methacrylate, alkylene dimethacrylates such as ethylene glycol dimethacrylate and propylene glycol dimethacrylate, hydroxyalkyl methacrylates such as hydroxyethyl methacrylate and hydroxypropyl methacrylate, pentaerythritol di-, tri- or tetramethacrylate trimethylol propane trimethacrylate or acrylate can be used.

As the acrylates, ethylene glycol diacrylates, pentaerythritol di-, tri- or tetra-acrylate can be used.

As the monovinyl monomer, styrene, substituted styrene (the substituent is a chloromethyl group, an alkyl group having up to 18 carbon atoms, a hydroxyl group, a t-butyloxycarbonyl group, a halogen group, a nitro group, an amino group, a protected hydroxyl group or amino group), vinyl naphthalene, acrylic acid esters, methacrylic acid esters, vinyl acetate and pyrrolidone, and mixtures thereof can be used.

As the monomer constituting the porous rigid polymeric self-supporting structure, preferablly methacrylates or acrylates, more preferably glycidyl methacrylate or alkylenedimethacrylates, and most preferably a copolymer of glycidyl methacrylate and ethylene glycol dimethacrylate which is modified with a compound having a butyl group, can be used.

This structure maintains the mechanical strength that can self-support its shape when left standing. That is, the physical structure is different from that using a bead-like porous polymer carrier or a porous membrane.

Specific examples of the porous rigid polymeric self-supporting structure include CIM (registered trademark) C4A DISK, CIM C4A-1 Tube Monolithic column, CIM C4A-8 Tube Monolithic column, CIM C4A-80 Tube Monolithic column, CIM C4A-800 Tube Monolithic column, CIM C4A-8000 Tube Monolithic column.

All of these are modified products of copolymers of glycidyl methacrylate and ethylene glycol dimethacrylate.

### <Method for Producing Rigid Porous Polymeric self-Supporting Structure>

The porous rigid polymeric self-supporting structure comprises a copolymer of the above-mentioned monomers. This copolymer is produced from a mixture of glycidyl methacrylate and ethylene glycol dimethacrylate in the presence of a porogen and a polymerization initiator.

A porogen is an additive substance for forming porosity, and it is an additive substance such as an aliphatic hydrocarbon, an aromatic hydrocarbon, an ester, an alcohol, a ketone, an ether, a soluble polymer solution, or different kinds of materials such a mixture thereof and the like can be used. Preferably, it is normal hexane.

As the polymerization initiator, a free radical generating initiator can be used. Specifically, azo compounds such as azobisisobutyronitrile and 2,2'-azobis (isobutylamide) dihydrate, and peroxides such as benzoyl peroxide and dipropyl dicarboxylic acid dicarboxylate can be used. Using different types of polymerization initiators, different pore structures can be formed. The amount of the polymerization initiator is preferably 0.5 to 4% by mass based on the monomer weight.

When preparing the copolymer, a soluble polymer may be added as a porogen. When a soluble polymer is added, more pore structure is formed. The amount of the soluble polymer is preferably from 10 to 40% by mass, based on the total mass of the copolymer.

It is preferable to degas a mixture of glycidyl methacrylate containing a porogen and a polymerization initiator and ethylene glycol dimethacrylate with an inert gas such as nitrogen or argon before placing it in a mold. The mold is preferably sealed to prevent air contamination.

The polymerization can be carried out, for example, at a temperature of 50°C to 90°C for 40 to 50 hours.

After polymerization, the tube is washed and solvent and soluble polymer used as porogen are removed. As a solvent for washing, methanol, ethanol, benzene, toluene, acetone, tetrahydrofuran and the like can be used. The washing step may be repeated a plurality of times.

Where the copolymer is modified, hydrophobic groups can be introduced using alcoholates such as sodium ethanolate, sodium butanolate, or sodium octanolate.

### <Column>

The column contains a rigid porous polymeric self-supporting structure with hydrophobic groups immobilized. That is, the column may be composed of only a porous rigid polymeric self-supporting structure in the form of a plate, a tube, a cylinder, or the like, and may be formed by disposing (supporting) a prescribed amount of a porous rigid polymeric self-supporting structure in a container in the form of a container. The storage container may have any structure as long as it has a structure allowing passage of liquid from the outside to the inside of the structure, and any material such as metal or resin may be used.

The column size (column volume) is not particularly limited, and is appropriately adjusted according to the amount of the protein aggregate and / or HCP to be adsorbed.

### <Protein>

The protein capable of removing the protein aggregate by the method of the first embodiment of the present invention is not particularly limited, and examples thereof include immunoglobulins such as immunoglobulin G (hereinafter referred to as "IgG"), immunoglobulin A (hereinafter referred to as "IgA"), immunoglobulin M (hereinafter referred to as "IgM"); cytokines such as interleukin, chemokine, interferon; G-CSF (Granulocyte-Colony Stimulating Factor), erythropoietin, EGF (Epidermal Growth Factor), FGF (hereinafter referred to as "FGF"), TGF (Transforming Growth Factor), BDNF (Brain - Derived Neurotrophic Factor), VEGF (Vascular Endothelial Growth Factor), GM - CSF (Granulocyte Macrophage Colony - Stimulating Factor), PDGF (Platelet - Derived Growth Factor), EPO (Erythropoietin), TPO (Thrombopoietin), bFGF (Fibroblast growth factors), HGF (Hepatocyte Growth Factor), TNF-α(Tumor Necrosis Factor Alpha), TGF-β(Transforming Growth Factor Beta), PAI-1(Plasminogen Activator Inhibitor-1), HB-EGF(Heparin-Binding EGF-Like Growth Factor), leptin, adiponectin, NGF (Nerve Growth Factor); protein hormones such as human growth hormone, insulin and glucagon; blood coagulation factors, albumin, lysozyme, RNase A (Ribonuclease A), cytochrome c and the like.

### <Protein monomer>

Protein monomer refers to one molecule of protein.

### <Protein aggregate>

Protein aggregates are protein complexes in which protein monomers are adsorbed and aggregated reversibly or irreversibly by hydrophobic interactions, electrostatic interactions, and other interactions.

### <HCP>

Host cell protein (HCP) is a protein derived from cells such as Escherichia coli, yeast, Chinese hamster ovary cells and mouse myeloma cells, which are used for manufacturing biopharmaceuticals. It is reported that some HCPs may contain proteins with immunogenicity and adjuvant like activity, and it is reported that the safety and stability of the formulations are deteriorated due to residual proteases and the like. Therefore, it is desirable to remove it.

### <Buffer solution>

The buffer solution is not particularly limited, but, for example, a phosphate buffer solution, a citrate buffer solution, a tris (trishydroxymethylaminomethane) buffer solution, an acetate buffer solution, a borate buffer solution and the like can be used. Among them, a phosphate buffer solution, a citrate buffer solution, and a Tris buffer solution are preferable from the viewpoint of the use pH range having a buffering ability.

Although the concentration of the buffer solution is not particularly limited, it is preferably 1 to 100 mM, more preferably 2 to 50 mM, most preferably 5 to 30 mM.

The pH of the buffer solution is not particularly limited, but is preferably pH 2 to 9, more preferably pH 3 to 8, further preferably pH 4 to 7.5.

### <Inorganic salt>

As the inorganic salt used for the salt-containing buffer solution, chaotropic salts and kosmotropic salts can be used, and it is preferable to use a kosmotropic salt. As the kosmotropic salt, specifically, phosphate ion, sulfate ion, acetate ion, chloride ion, bromide ion, nitrate ion, chlorate ion, iodide ion and thiocyanate ion can be used as anions. As a cation, ammonium ion, rubidium ion, potassium ion, sodium ion can be used.

The kosmotropic salt is preferably ammonium sulfate, sodium sulfate, potassium sulfate, ammonium phosphate, sodium phosphate, potassium phosphate, potassium chloride and sodium chloride, more preferably ammonium sulfate, sodium chloride.

The kosmotropic salt is preferably in an amount sufficient to adsorb most of the relevant impurities to the column. And it is sufficiently small so as not to cause binding or precipitation of protein monomers and protein aggregates.

Specifically, when ammonium sulfate is used as a salt, the concentration with respect to the buffer solution is preferably 500 to 950 mM, more preferably 600 to 900 mM, further preferably 700 to 800 mM. In the case of using sodium chloride as the salt, the concentration with respect to the buffer solution is preferably 0.5 to 4.0 M, more preferably 1.0 to 3.0 M, and further preferably 1.5 to 2.5 M.

For each purification process, it is preferable to select the optimum amount and preferred type of salt by preliminary experiment or the like.

### <Equilibration of column>

It is preferable to equilibrate column by passing the buffer through the column before loading the solution containing protein monomers and protein aggregates, and / or HCP onto the column.

As the type, concentration and pH of the buffer solution used for equilibration, the same buffer solution as that for dissolving the protein can be used. Equilibration may be carried out by a mixed solution of an inorganic salt and a buffer solution which will be described later.

Although the amount of buffer solution required for equilibration is not particularly limited, it is preferably 1 CV (column volume) or more, more preferably 2 CV or more, further preferably 4 CV or more.

### <Loading of protein monomers and protein aggregates and / or HCP on columns>

Protein monomer and protein aggregate, and / or HCP are dissolved in a buffer solution to prepare a solution (hereinafter this solution is referred to as "protein solution"), and this solution is passed through a column to make protein monomers and protein aggregates, and / or HCP to be loaded onto the column.

When dissolving a protein solution containing protein monomers and protein aggregates and / or HCP in a buffer solution, the concentration is preferably 0.001 to 20 mg/mL, more preferably 0.01 to 10 mg/mL, most preferably 0.1 to 5 mg/mL.

The protein loading amount per 1 mL of column volume is preferably 0.001 to 50 mg, more preferably 0.01 to 40 mg, further preferably 0.1 to 30 mg.

When loading the column with protein, the temperature of the column and the protein solution is not particularly limited, but is preferably 2 to 50°C, more preferably 4 to 40°C, most preferably 8 to 30°C. Within this range, freezing of the protein solution and destruction of the protein can be prevented.

### <Flow-Through of Protein Monomer>

Protein aggregates and / or HCP can be adsorbed to the column by passing the mobile phase through a column loaded with protein monomers and protein aggregates and / or HCP, and protein monomers can be recovered in a flow-through mode. As the solution of the mobile phase, a mixed solution obtained by mixing the salt-containing buffer solution in which the inorganic salt is dissolved and the buffer solution in an appropriate ratio can be used.

Note that the flow-through mode of protein monomer means that the desired product to be recovered is not adsorbed by the chromatography device but passes through it.

The type, concentration and pH of the buffer solution as the solution of the mobile phase may be the same as those of the buffer solution for dissolving the protein, and may be different, but it is desirable that they are the same.

The flow rates of the mobile phase and the protein solution are not limited as long as the object can be achieved, but can be set to 2 to 16 CV/min, preferably 2 to 12.5 CV/min, more preferably 2.5 to 5 CV / And more preferably 4 to 5 CV/min.

When passing the mobile phase through the column, the temperature of the column and the mobile phase is not particularly limited, but it is preferably 2 to 50°C, more preferably 4 to 40°C, still more preferably 8 to 30°C. Within this range, freezing of the protein solution and destruction of the protein can be prevented.

In the first embodiment of the present invention, the pH range of the buffer solution and protein solution is not limited. The first embodiment of the present invention may be carried out singly or after the purification step by ion exchange chromatography or affinity chromatography. Furthermore, the first embodiment of the present invention can be carried out without adding additives such as arginine prior to the purification step.

Therefore, the first embodiment of the present invention can obtain protein monomers in a high yield and high purity under more free conditions.

By examining the conditions for selectively recovering the protein monomers using the above findings, it is possible to shorten the time of condition examination. In particular, in the first embodiment of the present invention, even if the thickness of the column in the flowing direction or the diameter of the column is changed, the purification can be carried out without changing column condition such as the hydrophobic group, constituent monomer, temperature, pressure thereof; buffer solution; salt-containing buffer solution; the flow rate of the mobile phase (CV/min), so that the time of the examination condition can be shortened.

### <Elution of Protein Aggregate and / or HCP and Regeneration of Column>

After eluting the protein monomer by the above method, protein aggregates and / or HCP can be eluted, for example, by passing water through the column. After elution of the protein aggregate and / or HCP, the column can be regenerated by again passing through the same buffer as used for equilibration.

### (Second Embodiment: one-step Purification)

### [Method for Removing Protein Aggregate]

The method for removing protein aggregates according to the second embodiment of the present invention includes a step of recovering the protein monomer in a flow-through mode by absorbing the aggregate in the column by passing a protein solution, which contains a protein monomer and protein aggregates, through a column holding a porous rigid polymeric self-supporting structure to which a hydrophobic group is immobilized.

### [Removal method of protein aggregate and HCP]

The method for removing protein aggregate and HCP according to the second embodiment of the present invention includes a step of recovering the protein monomer in a flow-through mode by absorbing the aggregate and the host cell protein (HCP) in the column by passing a protein solution, which contains a protein monomer, the aggregate and a host cell protein (HCP), through a column holding a porous rigid polymeric self-supporting structure to which a hydrophobic group is immobilized.

A hydrophobic group, porous rigid polymeric self-supporting structure, column, protein, protein monomer, protein aggregate, HCP, buffer, and equilibration of column in the second embodiment of the present invention are the same as those in the first embodiment. As the same as that in the first embodiment of the present invention, the method for removing protein aggregates and HCPs according to the second embodiment of the present invention may further include a preliminary purification step by ion exchange chromatography, affinity chromatography, prior to the above-mentioned removing method.

### <Adsorption of Protein Aggregate and HCP and Flow-Through of Protein Monomer>

In the second embodiment of the present invention, after equilibration of the column, the step of" Loading of protein monomers and protein aggregates and / or HCP on columns " of the first embodiment of the present invention and the step of" Flow-Through of Protein Monomer " are combined in one step.

For example, a solution is prepared by dissolving protein monomers, protein aggregates and / or HCP in a buffer solution (hereinafter this solution is referred to as "protein solution"). Protein aggregates and HCP can be adsorbed on the column and protein monomers can be recovered in a flow-through mode by passing this solution through a column holding a porous rigid polymeric self-supporting structure to which a hydrophobic group is immobilized.

As the solution of the mobile phase, a mixed solution obtained by mixing the salt-containing buffer solution in which the inorganic salt is dissolved and the buffer solution in an appropriate ratio can be used.

In the second embodiment of the present invention, the buffer solution and the inorganic salt used for adsorption of protein aggregate and HCP and flow-through process of protein monomer are the same as those of the first embodiment.

When passing the mobile phase through the column, the temperature of the column and the protein solution is not particularly limited, but is preferably 2 to 50°C, more preferably 4 to 40°C, still more preferably 8 to 30°C. Within this range, freezing of the protein solution and destruction of the protein can be prevented.

The type, concentration and pH of the buffer solution as the mobile phase solution may be the same as, or different from those of the buffer solution used for equilibration of the column, but it is desirable that they are the same.

### <Elution of Protein Aggregate and / or HCP and Regeneration of Column>

In the second embodiment of the present invention, as in the first embodiment of the present invention, elution of protein aggregates and / or HCP and regeneration of the column can be carried out.

### EXAMPLE

Hereinafter, the effect of the present invention will be made clear by Examples. It should be noted that the present invention is not limited to the following examples, but can be carried out with appropriate modifications within the scope not changing the gist thereof.

In the examples, for example, when IgG is used as a protein, the recovery ratio of IgG monomer, the purity of IgG, and the content of IgG aggregate are defined as follows.

### <Recovery rate>

Recovery rate of IgG monomer is the ratio of IgG monomer recovered in a flow-through mode with respect to the total amount of IgG including whole amount of IgG recovered in a flow-through mode and whole amount of IgG adsorbed on the column. The whole amount of IgG is the sum of IgG monomer and IgG aggregate.

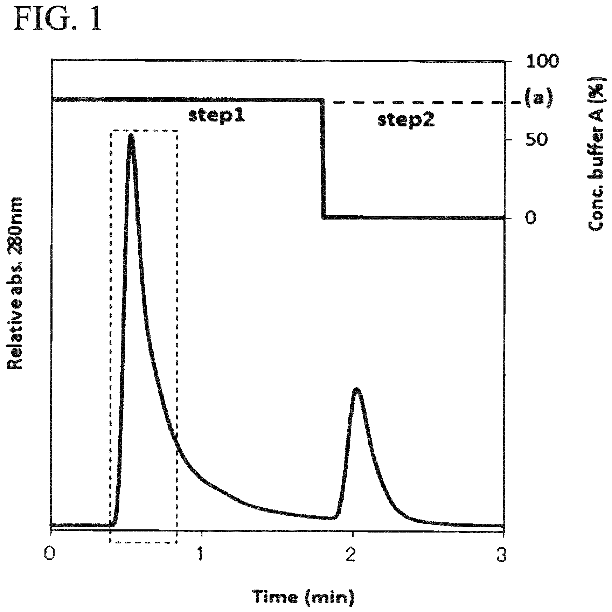

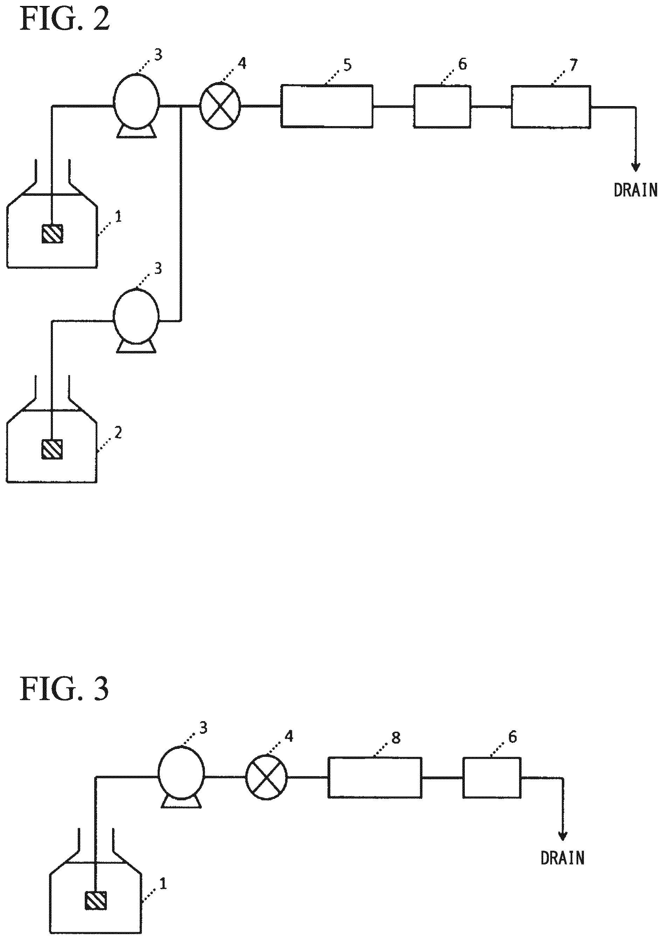

Specifically, the recovery rate is defined as the value obtained by dividing the area of the flow-through portion on the chromatogram by the sum of the area of the flow-through portion and the area of the elution portion by pure water. The flow-through portion is a peak portion surrounded by a dotted line in step 1 shown in FIG. 1, meaning a portion that passes through and is not adsorbed by the column. An HPLC apparatus (SCL-10 AVP) manufactured by Shimadzu Corporation was used for preparing the chromatogram. In this device, the buffer solution and the salt-containing buffer solution are connected to the auto-sampler via a liquid sending pump, and the concentration of the solution flowing in the column can be controlled. The flow path diagram is shown in FIG. 2. The unit is "%".

### <Purity>

The purity of recovered IgG monomer is the proportion of IgG monomer contained in the whole amount of IgG in the recovered sample. The whole amount of IgG is the sum of IgG monomer and IgG aggregate.

Specifically, the recovered amount in the flow-through is determined by measuring the size exclusion chromatography with Shodex (registered trademark) KW 403-4 F made by Showa Denko K.K. The purity is defined as the value obtained by dividing the peak area of the protein monomer on the chromatogram by the area of the whole amount of IgG. The area of the whole amount of IgG is the sum of the peak area of the protein aggregate and the peak area of the protein monomer. In order to prepare the chromatogram, an HPLC apparatus (LC20-AT) manufactured by Shimadzu Corporation was used. The flow path diagram is shown in FIG. 3. The unit is "%".

### <Protein aggregate content>

When a state where no protein aggregate is contained at all is defined as 100, the protein aggregate content is defined as 100 - purity (%). The purity (%) is calculated as the area ratio in the chromatogram.

Specifically, the recovered amount in the flow-through is determined by measuring the size exclusion chromatography with Shodex (registered trademark) KW 403-4 F made by Showa Denko K.K. The value obtained by dividing the peak area of protein aggregates on the chromatogram by the area of the whole amount of IgG is taken as the protein aggregate content. The area of the whole amount of IgG is the sum of the peak area of the protein aggregate and the peak area of the protein monomer. For preparing the chromatogram, an HPLC apparatus (LC20-AT) manufactured by Shimadzu Corporation was used. The flow path diagram is shown in FiG. 3. The unit is "%".

### <Example 1>

### (1) Equilibration of column

CIM (registered trademark) C4A-1 Tube (manufactured by BIA Separations; thickness: 6 mm, outer diameter: 18.6 mm, inner diameter: 6.7 mm, porosity: 60 v/v%, bed volume: 1. 0 mL) was passed over 5 CV of 15 mM citrate buffer (pH 4.3) containing 700 mM ammonium sulfate to be equilibrated. The CIM C4A-1 Tube is a modified product of a copolymer of glycidyl methacrylate and ethylene glycol dimethacrylate, and is a porous rigid polymeric self-supporting structure in which a butyl group is held in a part of glycidyl methacrylate. Also, since the hole in the center of the column is closed by the structure of the device, the injected liquid does not pass through the hole in the center of the column.

### (2) Load of IgG monomer and IgG aggregate

IgG (Reagent Grade, ≧ 95%, manufactured by Sigma-Aldrich) was dissolved in 15 mM citrate buffer (pH 4.3) containing 700 mM ammonium sulfate to adjust the concentration to 3 mg/mL. The protein aggregate content of this solution was approximately 5% with respect to the whole amount of IgG.

250 µL of this solution was injected into the column equilibrated at the step (1) and purified. That is, the protein loading amount per mL of column volume is 750 µg. The solution flow rate was 5 CV/min.

### (3) IgG monomer flow-through

250 µl of the solution which was mixed with 15 mM citrate buffer (pH 4.3) so that the ammonium sulfate concentration was 700 mM, was passed through, and the IgG monomer was recovered in a flow-through mode. The solution flow rate was 5 CV/min.

### (4) Elution of IgG aggregates

Thereafter, pure water was passed through a 7.5 ml column to elute IgG aggregates. The solution flow rate was 5 CV/min.

In Example 1, the temperature of the column, the protein solution, and the mobile phase was 25°C, and the pressure of the column was 0.3 to 1.8 MPa.

### <Example 2>

The example was carried out in the same method as in Example 1 except that the ammonium sulfate concentration in the step (3) of Example 1 was changed to 750 mM.

### <Example 3>

The example was carried out in the same method as in Example 1 except that the ammonium sulfate concentration in the step (3) of Example 1 was changed to 800 mM.

### <Example 4>

The example was carried out in the same method as in Example 1 except that the 15 mM citrate buffer solution (pH 4.3) of Example 1 was changed to 15 mM phosphate buffer solution (pH 7.0).

### <Example 5>

The example was carried out in the same method as in Example 4 except that the concentration of ammonium sulfate in the step (3) of Example 4 was 750 mM.

### <Example 6>

The example was carried out in the same method as in Example 4 except that the concentration of ammonium sulfate in the step (3) of Example 4 was changed to 800 mM.

### <Example 7>

### (1) Equilibration of column

This was carried out in the same method as in the step (1) of Example 1 except that 700 mM ammonium sulfate was changed to 2.4 M sodium chloride and the pH of the citrate buffer was changed to 5.3.

### (2) Load of IgG monomer and IgG aggregate

The example was the same as in the step (2) of Example 1 except that 700 mM ammonium sulfate was changed to 2.2 M sodium chloride and the pH of the citrate buffer was changed to 5.3.

### (3) IgG monomer flow-through

The example was the same as in the step (3) of Example 1 except that the pH of the citrate buffer was 5.3 and the solution mixed with the buffer so that the sodium chloride concentration became 2.4 M was passed through.

### (4) Elution of IgG aggregates

The example was carried out in the same method as in the step (4) of Example 1.

### <Example 8>

The example was carried out in the same method as in Example 7 except that the sodium chloride concentration in the step (3) of Example 7 was changed to 2.2 M.

### <Example 9>

The example was carried out in the same method as in Example 8 except that the concentration of sodium chloride in the step (3) of Example 7 was changed to 2.0 M.

### <Example 10>

The example was carried out in the same method as in Example 1 except that the protein loading amount per 1 mL of the column volume of the step (2) of Example 1 was changed to 38 mg.

In Example 10, the column pressure was 0.3 to 1.8 MPa.

### <Example 11>

### (1) Equilibration of column

The example was carried out in the same method as in the step (1) of Example 1 except that CIM (registered trademark) C4A DISK (thickness: 3 mm, diameter: 12 mm, porosity: 60 v/v%) was used as the column and 20 mM phosphate buffer (pH7.0) was used as the buffer. CIM C4A DISK was a modified product of a copolymer of glycidyl methacrylate and ethylene glycol dimethacrylate, and was a porous rigid polymeric self-supporting structure in which a butyl group is held in a part of glycidyl methacrylate.

### (2) Load of IgG monomer and IgG aggregate

The example was carried out in the same method as in the step (2) of Example 1 except that the buffer solution was 20 mM phosphate buffer (pH 7.0), the concentration of IgG was 1 mg/mL, the load amount of protein per mL of column volume was 294 µg, and the flow rate of the solution was 4 CV.

### (3) IgG monomer flow-through

The example was carried out in the same method as in the step (3) of Example 1 except that the buffer solution was 20 mM phosphate buffer (pH 7.0).

### (4) Elution of IgG aggregates

The example was carried out in the same method as in the step (4) of Example 1.

In Example 11, the column pressure was 0.3 to 1.8 MPa.

### <Comparative Example 1>

### (1) Equilibration of column

The example was carried out in the same method as in the step (1) of Example 1 except that CIMmlutus (registered trademark) SO3-1 mL Advanced Composite Column (thickness: 6 mm, outer diameter: 18.6 mm, inner diameter: 6.7 mm, porosity: 60 v/v%) was used as a column, 150 mM sodium chloride was used instead of 700 mM ammonium sulfate, the concentration of the buffer solution was 20 mM, and the pH was 5.3. The CIMmlutus SO3-1 mL Advanced Composite Column was a modified product of a copolymer of glycidyl methacrylate and ethylene glycol dimethacrylate, and a porous rigid polymeric self-supporting structure in which sulfo groups are retained in some glycidyl methacrylate.

### (2) Load of IgG monomer and IgG aggregate

The example was carried out in the same method as in the step (2) of Example 1 except that 150 mM sodium chloride was used instead of 700 mM ammonium sulfate, buffer concentration was 20 mM, pH was 5.3 IgG, concentration was 3 mg/mL, load amount of protein per mL column volume was 450 µg, and solution flow rate was 5 CV.

### (3) IgG monomer flow-through

The experiment was carried out in the same method as in the step (3) of Example 1 except that the concentration of the buffer solution was 20 mM, the pH was 5.3, and a solution mixed with the buffer solution so that the sodium chloride concentration became 150 mM.

### (4) Elution of IgG aggregates

The example was carried out in the same method as in the step (4).

The conditions and results of the examples and comparative examples are summarized in Table 1.

In Examples 1 to 11 which are the first embodiment of the present invention, although the type and thickness of the column, IgG loading amount, flow rate, pH, and salt type were changed, it was possible to improve the recovery rate and IgG monomer purity.

On the other hand, in Comparative Example 1 using a column having an anion exchange group, both the recovery rate and IgG monomer purity decreased.

From the above, the superiority of the first embodiment of the present invention could be confirmed.

In Examples 1 to 11, even in the case of a high flow rate of 5 CV/min which cannot be reached by using a bead-like porous polymer carrier or a porous membrane, it is possible to obtain a protein monomer with high recovery rate and high purity. In the column used in Comparative Example 1, although the recovery rate and the purity could be improved by lowering the flow rate, in the case of 5 CV/min, which is equivalent to the Examples, the recovery rate and purity decreased.

From this, the superiority of the first embodiment of the present invention at high flow velocity could be confirmed.

**Table 1**

| | Column | | | | (3) Mixed solution | | Buffer solution | | | | Flow rate at Flow-through Elution [CV/min] | Recovery rate [%] | purity [%] |
|---|---|---|---|---|---|---|---|---|---|---|---|---|---|
| | Type | Constitution Monomer | Hydrophobic group | Thickness [mm] | Salt | Salt concentration | Type | Concentration [mM] | pH | Loading amount | | | |
| Example 1 | CIM C4A-1 Tube | Glycidyl Methacrylate Ethylene Glycol Dimethacrylate | Butyl group | 6 | Ammonium sulfate | 700 mM | Citric acid | 15 | 4.3 | 750 µg | 5 | 84.6 | 99.1 |
| Example 2 | CIM C4A-1 Tube | | Butyl group | 6 | Ammonium sulfate | 750 mM | Citric acid | 15 | 4.3 | 750 µg | 5 | 79.4 | 99.5 |
| Example 3 | CIM C4A-1 Tube | | Butyl group | 6 | Ammonium sulfate | 800 mM | Citric acid | 15 | 4.3 | 750 µg | 5 | 71.6 | 100 |
| Example 4 | CIM C4A-1 Tube | | Butyl group | 6 | Ammonium sulfate | 700 mM | Phosphoric acid | 15 | 7.0 | 750 µg | 5 | 76.7 | 96.7 |
| Example 5 | CIM C4A-1 Tube | | Butyl group | 6 | Ammonium sulfate | 750 mM | Phosphoric acid | 15 | 7.0 | 750 µg | 5 | 71.4 | 97.9 |
| Example 6 | CIM C4A-1 Tube | | Butyl group | 6 | Ammonium sulfate | 800 mM | Phosphoric acid | 15 | 7.0 | 750 µg | 5 | 64.1 | 99 |
| Example 7 | CIM C4A-1 Tube | | Butyl group | 6 | Sodium chloride | 2.4 M | Citric acid | 15 | 5.3 | 750 µg | 5 | 74.9 | 99.3 |
| Example 8 | CIM C4A-1 Tube | | Butyl group | 6 | Sodium chloride | 2.2 M | Citric acid | 15 | 5.3 | 750 µg | 5 | 69.5 | 99.6 |
| Example 9 | CIM C4A-1 Tube | | Butyl group | 6 | Sodium chloride | 2M | Citric acid | 15 | 5.3 | 750 µg | 5 | 57.8 | 99.9 |
| Example 10 | CIM C4A-1 Tube | | Butyl group | 6 | Ammonium sulfate | 700 mM | Citric acid | 15 | 4.3 | 38 mg | 5 | 93.6 | 94.3 |
| Example 11 | CIM C4A DISK | | Butyl group | 3 | Ammonium sulfate | 700 mM | Phosphoric acid | 20 | 7.0 | 294 µg | 4 | 81.0 | 98.1 |
| Example 12 | CIM C4A-1 Tube | | Butyl group | 6 | Ammonium sulfate | 750 mM | phosphoric acid | 20 | 4.3 | 50 mg | 5 | 95.6 | 99.5 |
| Comparative Example 1 | CIMmltus S O3-1mL Advanced Composite Column | | None (Functional group: Sulfo group) | 6 | Sodium chloride | 150 mM | Citric acid | 20 | 5.3 | 450 µg | 5 | 69.0 | 96.2 |
| Comparative Example 2 | HiTrap SP FF | Beads | A sulfo group | 6 | Ammonium sulfate | 750 mM | phosphoric acid | 20 | 4.3 | 50 mg | 1 | 63 | 94 |

### <Example 12>

### (1) Equilibration of column

CIM (registered trademark) C4A-1 Tube manufactured by BIA separations was equilibrated with 5 CV or more of 15 mM citrate buffer (pH 4.3) containing 750 mM ammonium sulfate.

### (2) Adsorption of IgG aggregate and HCP and flow-through of IgG monomer

A monoclonal antibody solution (20 mM phosphate buffer (pH 7.4)) after chromatography on a protein A column and an anion exchange column was adjusted to pH 4.3 with 1 M citric acid solution containing 750 mM ammonium sulfate. The content of protein aggregates in the total amount of protein monomers and protein aggregates of this solution was approximately 5%. In this solution the concentration of protein containing IgG monomer, IgG aggregate and HCP was approximately 1 mg/mL.

50 mL of this solution was injected into the column equilibrated with the step (1), and the IgG aggregate and HCP were adsorbed to allow flow-through of the IgG monomer. That is, the loading amount of monoclonal antibody per 1 mL of the column volume was about 50 mg. The solution flow rate was 5 CV/min.

### (3) Elution of IgG aggregate and HCP

Thereafter, 7.5 mL pure water was passed through the column to elute IgG aggregates and HCP. The solution flow rate was 5 CV/min.

HCP quantitative evaluation was performed using CHO Host Cell Proteins 3rd Generation kit (manufactured by CYGNUS). The amount of HCP before passing through the column was about 0.008 µg / mL, but it was about 0.0002 µg / mL after passing through the column, and the removal rate was about 98%.

### <Comparative Example 2>

The example was carried out in the same method as in Example 12 except that HiTrap SP FF (average particle diameter 90 µm) manufactured by GE Healthcare Co., Ltd. was used as the porous polymer beads constituting the column and the flow rate of the protein solution in the column was 1 CV/min. As a result, the IgG monomer was selectively eluted.

The conditions and results of Example 12 and Comparative Example 2 are summarized in Table 1.

In addition, in Example 12, a protein monomer was obtained with a high recovery rate and purity even in the case of a high flow rate of 5 CV/min which cannot be reached when using a bead-like porous polymer carrier or a porous membrane. On the other hand, in Comparative Example 2 using the porous polymer beads, it was possible to recover the IgG monomer while suppressing the flow rate, but both the recovery rate and the purity did not reach Example 12. Also, from the viewpoint of permissible pressure, it was not possible to increase the flow rate to 2 CV/min or more. Therefore, the superiority in the high flow velocity of the second embodiment of the present invention was confirmed.

### Explanation of Signs

1: Buffer solution
2: Salt containing buffer
3: Feed pump
4: Autosampler
5: Preparative column
6: PDA detector
7: Fraction collector
8: Analytical column

## Claims

1. A protein purification method, comprising a step of recovering a protein monomer in a flow-through mode by passing a protein solution, which contains the protein monomer and a protein aggregate, through a column holding a porous rigid polymeric self-supporting structure to which a hydrophobic group is immobilized.

2. The purification method according to claim 1, comprising steps of loading a protein monomer and a protein aggregate by passing a solution, which contains the protein monomer and the aggregate, through a column holding a porous rigid polymeric self-supporting structure to which a hydrophobic group is immobilized, and
recovering the protein monomer in a flow-through mode by absorbing the aggregate by passing a mobile phase through the column loaded with the protein monomer and the protein aggregate.

3. A purification method according to claim 1, comprising a step of recovering the protein monomer in a flow-through mode by absorbing the aggregate in the column by passing a protein solution, which contains a protein monomer and protein aggregates, through a column holding a porous rigid polymeric self-supporting structure to which a hydrophobic group is immobilized.

4. The purification method according to claim 1, wherein the protein solution further contains a host cell protein (HCP).

5. The purification method according to claim 4, comprising steps of loading a protein monomer, the aggregate and a host cell protein (HCP) by passing a solution, which contains the protein monomer, the aggregate and a host cell protein (HCP), through a column holding a porous rigid polymeric self-supporting structure to which a hydrophobic group is immobilized, and
recovering the protein monomer in a flow-through mode by absorbing the aggregate and a host cell protein (HCP) by passing a mobile phase through the column loaded with the protein monomer, the aggregate and a host cell protein (HCP).

6. The purification method according to claim 4, comprising a step of recovering the protein monomer in a flow-through mode by absorbing the aggregate and the host cell protein (HCP) in the column by passing a protein solution, which contains a protein monomer, the aggregate and a host cell protein (HCP), through a column holding a porous rigid polymeric self-supporting structure to which a hydrophobic group is immobilized.

7. The purification method according to any one of claims 1 to 6, wherein the monomer constituting the porous rigid polymeric self-supporting structure is a methacrylate or an acrylate.

8. The purification method according to any one of claims 1 to 7, wherein the thickness of the porous rigid polymeric self-supporting structure in the liquid flowing direction is 1 to 100 mm.

9. The purification method according to any one of claims 1 to 8, wherein the flow rate of the mobile phase is 2 CV/min or more.

10. The purification method according to any one of claims 1 to 9, wherein the concentration of the protein contained in the protein solution is 0.001 to 20 mg/mL.

11. The purification method according to any one of claims 1 to 10, wherein the protein loading amount per 1 mL of the column volume of the column is 0.001 to 50 mg.

12. The purification method according to any one of claims 1 to 11, further comprising a preliminary purification step using an ion exchange chromatography, and an affinity chromatography, and
the purification method is carried out after the preliminary purification step.
